(19) Europäisches Patentamt
European Patent Office
Office européen des brevets

(11) Veröffentlichungsnummer : **0 421 561 B1**

(12) # EUROPÄISCHE PATENTSCHRIFT

(45) Veröffentlichungstag der Patentschrift : **06.04.94 Patentblatt 94/14**

(51) Int. Cl.$^5$ : **C07C 401/00,** A61K 31/59

(21) Anmeldenummer : **90250249.1**

(22) Anmeldetag : **01.10.90**

(54) **24-Homo-Vitamin-D-Derivate, Verfahren zu ihrer Herstellung; diese Derivate enthaltende pharmazeutische Präparate sowie deren Verwendung als Arzneimittel.**

(30) Priorität : **02.10.89 DE 3933034**

(43) Veröffentlichungstag der Anmeldung : **10.04.91 Patentblatt 91/15**

(45) Bekanntmachung des Hinweises auf die Patenterteilung : **06.04.94 Patentblatt 94/14**

(84) Benannte Vertragsstaaten : **AT BE CH DE DK ES FR GB GR IT LI LU NL SE**

(56) Entgegenhaltungen :
**EP-A- 0 245 524**
**WO-A-86/02078**
**WO-A-89/10353**

(73) Patentinhaber : **SCHERING AKTIENGESELLSCHAFT**
**Müllerstrasse 170/178 Postfach 65 03 11**
**D-13303 Berlin (DE)**

(72) Erfinder : **Kirsch, Gerald, Dr.**
**Luciusstrasse 60**
**D-1000 Berlin 33 (DE)**
Erfinder : **Neef, Günter, Dr.**
**Markgraf-Albrecht-Strasse 4**
**D-1000 Berlin 15 (DE)**
Erfinder : **Schwarz, Katica**
**Zillestrasse 109**
**D-1000 Berlin 10 (DE)**
Erfinder : **Bräutigam, Matthias, Dr.**
**Droysenstrasse 8**
**D-1000 Berlin 12 (DE)**
Erfinder : **Thieroff-Eckerdt, Ruth, Dr.**
**Am Vierrutenberg 47**
**D-1000 Berlin 28 (DE)**
Erfinder : **Rach, Petra**
**Antwerpener Strasse 1**
**D-1000 Berlin 65 (DE)**

**EP 0 421 561 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 24-Homo-Vitamin-D-Derivate der Formel I

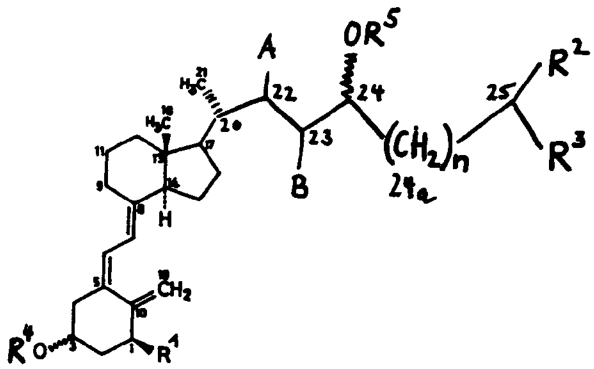

(I),

worin

| | |
|---|---|
| $R^1$ | ein Wasserstoffatom, eine Hydroxy- oder eine Acyloxy-gruppe mit 1 bis 8 Kohlenstoffatomen, |
| $R^2$ und $R^3$ | unabhängig voneinander eine 1 bis 4 Kohlenstoffatome aufweisende lineare oder verzweigte Alkylgruppe, eine Trifluormethylgruppe oder gemeinsam einen mit dem Kohlenstoffatom 25 gebildeten gesättigten carbocyclischen Ring mit 3 bis 9 Kohlenstoffatomen |
| $R^4$ und $R^5$ | unabhängig voneinander ein Wasserstoffatom oder 1 bis 8 Kohlenstoffatome aufweisende Acylgruppe und |
| A und B | jeweils ein Wasserstoff oder gemeinsam eine zweite Bindung bedeuten sowie |
| n | für 1, 2, 3, 4 oder 5 |

steht, sowie ein Verfahren zu deren Herstellung, pharmazeutische Präparate, die diese Verbindungen enthalten sowie deren Verwendung zur Herstellung von Arzneimitteln.

Die für die Reste $R^1$, $R^4$ und $R^5$ möglichen Acyloxy- bzw. Acylgruppen sind insbesondere von gesättigten Carbonsäuren oder auch der Benzoesäure abgeleitet.

Bilden $R^2$ und $R^3$ gemeinsam mit dem Kohlenstoffatom 25 einen gesättigten carbocyclischen Ring, so ist insbesondere an den Cyclopropyl-, Cyclopentyl- oder Cyclohexylring gedacht.

Bevorzugt gemäß vorliegender Erfindung sind 24-Homo-Vitamin-D-Derivate der allgemeinen Formel I, in welchen

$R^1$ für eine Hydroxygruppe oder

$R^2$ und $R^3$ für eine Methylgruppe, Ethylgruppe oder gemeinsam mit dem Kohlenstoffatom 25 für einen Cyclopropyl-, Cyclopentyl- oder Cyclohexylring oder

$R^4$ und $R^5$ für ein Wasserstoffatom

steht/stehen und n 1, 2 oder 3 ist.

Zwischen den Kohlenstoffatomen 22 und 23 befindet sich vorzugsweise eine Doppelbindung.

Besonders bevorzugt sind die Verbindungen

(5Z,7E,22E)-(1S,3R,24R)-9,10-Seco-24a-homo-5,7,10(19),22-cholestatetraen-1,3,24-triol und

(5Z,7E,22E)-(1S,3R,24S)-9,10-Seco-24a-homo-5,7,10(19),22-cholestatetraen-1,3,24-triol.

Die natürlichen Vitamine $D_2$ und $D_3$ bzw. deren biologisch aktive Metaboliten (die natürlichen Vitamine $D_2$ und $D_3$ sind an sich biologisch inaktiv und werden erst nach Hydroxylierung in 25-Position in der Leber bzw. in 1-Position in der Niere aktiv) weisen als charakteristisches Strukturmerkmal eine Doppelbindung zwischen den Positionen $C_{10}$ und $C_{19}$ auf, die als entscheidend für die Vitamin D-Aktivität angesehen wird (vgl. allgemeine Formel V). Die Wirkung der Vitamine $D_2$ und $D_3$ besteht in der Stabilisierung des Plasma-$Ca^{++}$- und Plasma-Phosphat-Spiegels; sie wirken einem Absinken des Plasma-$Ca^{++}$-Spiegels entgegen.

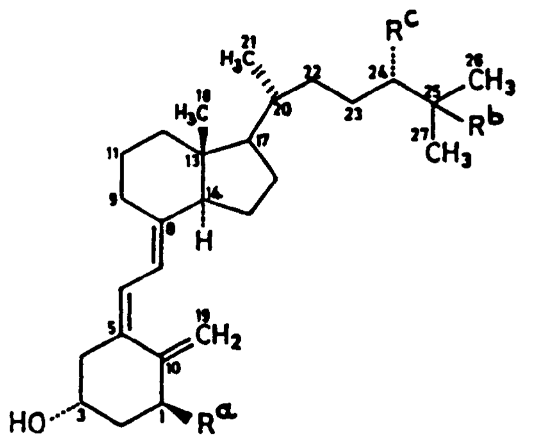

(V)

Ergocalciferol: $R^a = R^b = H$, $R^c = CH_3$, Doppelbindung C-22/23 — Vitamin $D_2$

Cholecalciferol: $R^a = R^b = R^c = H$ — Vitamin $D_3$

25-Hydroxycholecalciferol: $R^a = R^c = H$, $R^b = OH$

1α-Hydroxycholecalciferol: $R^a = OH$, $R^b = R^c = H$

1α,25-Dihydroxycholecalciferol: $R^a = R^b = OH$, $R^c = H$ — Calcitriol

Neben ihrer ausgeprägten Wirkung auf den Calcium- und Phosphatstoffwechsel besitzen Vitamin $D_2$ und $D_3$ und seine synthetischen Abkömmlinge auch proliferationshemmende und zelldifferenzierende Wirkungen (H.F. De Luca, The Metabolism and Function of Vitamin D in Biochemistry of Steroid Hormones, Hrsg. H.L.J. Makin, 2nd Edition, Blackwell Scientific Publications 1984, S. 71-116). Bei Vitamin D-Anwendung kann es aber zu Überdosierungserscheinungen kommen (Hypercalcämie).

In 24-Stellung hydroxylierte 1α-Cholecalciferole gehen bereits aus der DE-AS-25 26 981 hervor; sie besitzen eine geringere Toxizität als das entsprechende nicht-hydroxylierte 1α-Cholecalciferol. Die hydroxylierten Verbindungen zeigen eine selektive Aktivierung der intestinalen Calciumabsorption und eine schwächere Knochenabsorptionswirkung als 1α-Cholecalciferol.

Die in der internationalen Patentanmeldung WO 87/00834 beschriebenen 24-Hydroxy-Vitamin-D-Analoga können für die Behandlung von durch abnormer Zellproliferation und/oder Zelldifferentiation hervorgerufenen Störungen beim Menschen und Tier dienen.

Für verschiedene 1,25-Dihydroxy-Homo-Vitamin-D-Derivate ist eine Dissoziation bezüglich der Eigenschaften Knochenabsorptionswirkung und HL-60 Zelldifferentiation schon kürzlich von De Luca erwähnt worden. Die Knochenabsorptionswirkung in vitro ist dabei ein direktes Maß für die Calciummobilisierung in vivo.

Es wurde nun gefunden, das die erfindungsgemäßen 24-Homo-Vitamin-D-Derivate der allgemeinen Formel I im Vergleich zum Vitamin-D-Abkömmling Calcitriol (1α,25-Dihydroxycholecalciferol) überraschenderweise ein günstigeres Wirkungsspektrum aufweisen. Während die Effekte auf den Calcium- und Phosphatstoffwechsel deutlich abgeschwächt sind (Verringerung der Nebenwirkungen durch Überdosierung oder erforderlicher hoher Dosierung), bleiben die proliferationshemmenden und zelldifferenzierenden Wirkungen annähernd erhalten (Dissoziation).

Die Vitamin-D-Aktivität der erfindungsgemäßen Verbindungen wird mittels des Calcitriol-Rezeptortests bestimmt. Er wird unter Verwendung eines spezifischen Rezeptorproteins aus dem Darm rachitischer Hühner durchgeführt.

Rezeptorhaltiges Bindungsprotein wird mit $^3H$-Calcitriol (0,5 ng/ml) in einem Reaktionsvolumen von 0,575 ml in Abwesenheit und in Anwesenheit der Prüfsubstanzen für eine Stunde in einem Teströhrchen inkubiert. Zur Trennung von freiem und rezeptorgebundenem Calcitriol wird eine Charcoal-Dextran-Absorption durchgeführt. Dazu werden 200 μl einer Charcoal-Dextran-Suspension jedem Teströhrchen zugeführt und bei 22°C für 30 Minuten inkubiert. Anschließend werden die Proben bei 1500 x g 10 Minuten bei 4°C zentrifugiert. Der Überstand wird dekantiert und nach ca. 1stündiger Äquilibrierung in Atom-Light in einem β-Zähler gemessen.

Die mit verschiedenen Konzentrationen der Prüfsubstanz sowie der Referenzsubstanz (unmarkiertes Calcitriol) erhaltenen Kompetitionskurven für die Verdrängung von $^3H$-markierter Referenzsubstanz werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Demnach besitzt

(5Z,7E,22E)-(1S,3R,24R)-9,10-Seco-24a-homo-5,7,10(19),22-cholestatetraen-1,3,24-triol (Verbindung A) einen KF-Wert von 67 und

(5Z,7E,22E)-(1S,3R,24S)-9,10-Seco-24a-homo-5,7,10(19),22-cholestatetraen-1,3,24-triol (Verbindung B) einen KF-Wert von 0,8.

Zur Bestimmung der antiproliferativen Potenz der erfindungsgemäßen Verbindungen wird stellvertretend mit den Verbindungen A und B als Prüfsubstanzen der nachfolgend beschriebene Test durchgeführt:

Keratinocyten von neugeborenen Mäusen werden in Abwandlung der Methode von Yuspa, S. und Harris, C.C., "Altered differentiation of mouse epidermal cells treated with retinyl acetate in vitro", Exp. Cell Res. 86:95-105, 1974 präpariert und kultiviert.

Neonatale NMRI-Mäuse beiderlei Geschlechts werden durch Dekapitation getötet, die Haut abpräpariert, in einer Antibiotica-Antimykotika-Lösung gewaschen und mit der dermalen Seite nach unten in Dispase II-Lösung (1,2U/ml in Gewebekulturmedium M199 +25 mmol/l HEPES + 15% fötales Kälberserum (FCS) + 50 U/ml Penicillin/Streptomycin (P/S) (Präparationsmedium, PM) bei 4°C über Nacht inkubiert. Die Epidermis wird abgezogen und durch Trypsinierung eine Einzelzellsuspension hergestellt. Nach Zentrifugation wird das Zellsediment resuspendiert, nach Trypanblaufärbung die Zahl lebender kleiner runder Zellen bestimmt und die Zellen in einer Dichte von $4x10^5$ Zellen /$cm^2$ in Primaria-24-Loch-Platten in Gewebekulturmedium (M199 + 15% FCS + 50 U/ml P/S) ausgesät. Nach 24 Stunden Inkubation bei 37°C werden die Zellen mit phosphatgepufferter Salzlösung (PBS) gewaschen und weitere 24 Stunden in serumfreiem Gewebekulturmedium (M199 + 50 U/ml P/S + 0,5% Ethanol) mit und ohne Testsubstanzen bei 32,5°C inkubiert. Dann werden 0,4 µCi/50µl $^3$H-Methylthymidin (40Ci/mmol) zugegeben.Nach 4 Stunden wird das Medium abgesaugt und die Reaktion durch Zugabe von 500 µl eiskalter 10%iger Trichloressigsäure (TCA) beendet. Die Zellen werden mit TCA und PBS gewaschen, durch Inkubation in einer Proteinase K-Lösung (10 mmol/l Tris-HCl, 10 mmol/l EDTA, 10 mmol/l NaCl, 0.2% Triton-X 100, pH 8,0, 50 µg/ml Proteinkinase K) lysiert und das Lysat durch Zentrifugation geklärt. Im Überstand wird szintillationsphotometrisch die Radioaktivität und, nach spezifischer Färbung der DNA mit Diamidinophenylindol (DAPI), die DNA-Konzentration fluoreszenzphotometrisch bestimmt.

Demnach hemmen Calcitriol sowie die Verbindungen A und B dosisabhängig den $^3$H-Thymidin-Einbau in DNA mit annähernd denselben $IC_{50}$-Werten:

| | |
|---|---|
| Calcitriol | $2{,}7 \times 10^{-9}$ mol/l |
| Verbindung A | $6{,}0 \times 10^{-9}$ mol/l |
| Verbindung B | $9{,}5 \times 10^{-9}$ mol/l |

Durch das verminderte Hypercalciämie-Risiko eignen sich die erfindungsgemäßen Substanzen in besonderer Weise zur Herstellung von Arzneimitteln für die Behandlung von Erkrankungen, die durch eine Hyperproliferation gekennzeichnet sind, z. B. hyperproliferative Erkrankungen der Haut (Psoriasis) und maligne Tumoren (Leukämie, Coloncarcinom, Mammacarcinom). Voraussetzung für eine erfolgreiche Behandlung ist der Nachweis von Calcitriolrezeptoren im Zielorgan.

Die vorliegende Erfindung bezieht sich somit auch auf pharmazeutische Präparate, die mindestens eine Verbindung gemäß der allgemeinen Formel I zusammen mit einem pharmazeutisch verträglichen Träger enthalten. Die Verbindungen können formuliert werden als Lösungen in pharmazeutisch verträglichen Solventien oder als Emulsionen, Suspensionen oder Dispersionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nicht-toxische Hilfsstoffe enthalten, wie z. B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch on der Form von Cremes, Salben, Lotions oder geeigneter transdermaler Pflaster appliziert, wie in der EP-A-0387 077 beschrieben ist.

Die tägliche Dosis liegt bei

0,1 µg/Patient/Tag - 1000 µg (1 mg)/Patient/Tag,

vorzugsweise

1,0 µg/Patient/Tag - 500 µg/Patient/Tag.

Außerdem betrifft die Erfindung die Verwendung der Verbindungen gemäß Formel I zur Herstellung von Arzneimitteln.

Die Herstellung der 24-Homo-Vitamin-D-Derivate der Formel I erfolgt erfindungsgemäß dadurch, daß eine Verbindung der allgemeinen Formel IV

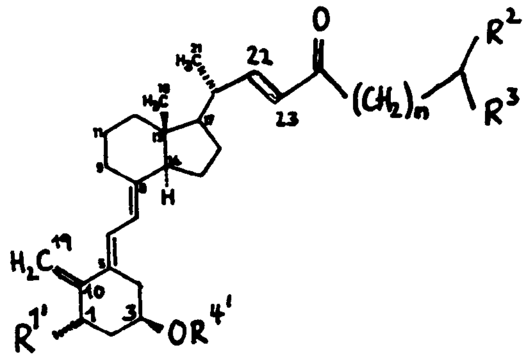

(IV),

worin
$R^{1'}$ ein Wasserstoffatom oder eine geschützte Hydroxygruppe und
$R^{4'}$ eine Hydroxyschutzgruppe bedeuten und
$R^2$ und $R^3$ sowie n die in Formel I angegebene Bedeutung haben, gegebenenfalls nach selektiver Hydrierung der 22,23-Doppelbindung, durch Reduktion der 24-Carbonylfunktion in eine Verbindung der allgemeinen Formel III

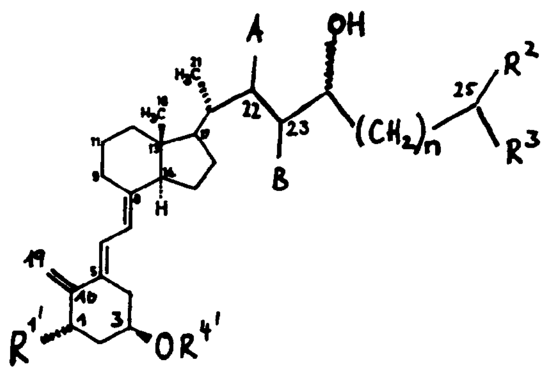

(III),

worin $R^{1'}$, $R^{4'}$, $R^2$ und $R^3$ sowie n die in Formel IV angegebene Bedeutung haben und
A und B entweder gemeinsam eine zweite Bindung oder jeweils ein Wasserstoffatom bedeuten
überführt,
eine Verbindung der allgemeinen Formel III durch Bestrahlung mit ultraviolettem Licht unter Umkehr der Stereoisomerie an der 5,6-Doppelbindung in eine Verbindung der allgemeinen Formel II

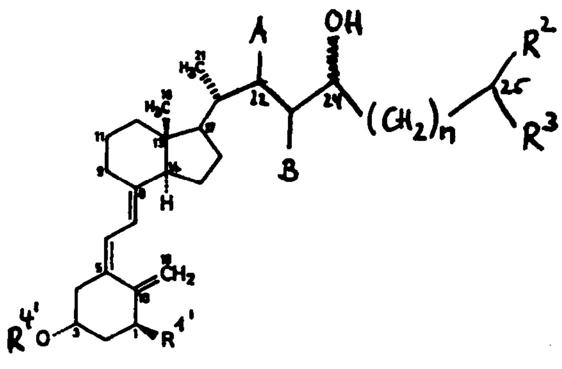

(II),

worin
$R^{1'}$, $R^{4'}$, $R^2$ und $R^3$, n sowie A und B die in Formel III angegebene Bedeutung haben,
umgewandelt
und diese anschließend durch Abspaltung vorhandener Hydroxyschutzgruppen und gegebenenfalls durch partielle oder vollständige Veresterung der Hydroxygruppen in eine Verbindung der allgemeinen Formel I überführt wird.

Die Reduktion der 24-Carbonylfunktion in der Verbindung der allgemeinen Formel IV erfolgt beispielsweise mit Cer(III)chlorid/Natriumborhydrid in einem polaren Solvens. Bei der Reduktion entsteht sowohl das (24R)- als auch das (24S)-24-Hydroxyisomere der allgemeinen Formel III. Die beiden Isomeren lassen sich chromatographisch trennen.

Gewünschtenfalls kann vor Redukion der Carbonylfunktion die C22,C23-Doppelbindung selektiv hydriert werden.

Die nachfolgende Umwandlung einer Verbindung der allgemeinen Formel III in eine Verbindung der allgemeinen Formel II erfolgt z.B. durch Bestrahlung mit ultraviolettem Licht in Gegenwart eines sogenannten "Triplettsensibilisators". Im Rahmen der vorliegenden Erfindung wird hierfür Anthracen verwendet. Durch Spaltung der $\pi$ -Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt.

Anschließend werden vorhandene Hydroxyschutzgruppen abgespalten, vorzugsweise unter Verwendung von Tetra-n-butyl-ammoniumfluorid sowie gewünschtenfalls die freien Hydroxygruppen nach gängigen Verfahren partiell oder vollständig mit dem entsprechenden Carbonsäurehalogenid (Halogenid=Chlorid, Bromid) oder Carbonsäureanhydrid verestert.

Die Herstellung der Ausgangsverbindungen der allgemeinen Formel IV geht aus von den in Tetrahedron 43, 4609(1987) bzw. in der internationalen Patentanmeldung WO 87/00834 von Calverley et al. beschriebenen Verbindungen des Typs

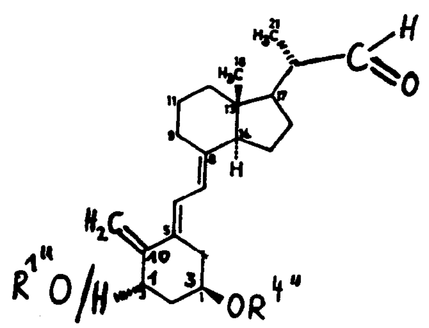

worin für die Hydroxyschutzgruppen $R^{1''}$ und $R^{4''}$ der Dimethyl-tert. -butylsilylrest steht; auch andere Trialkylsilylreste sind als Schutzgruppen erfindungsgemäß denkbar.
Umsetzung mit einem Phosphoran der Formel VI

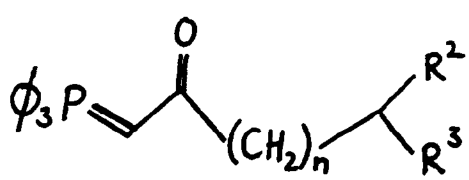

(VI)

führt zu Verbindungen der allgemeinen Formel IV (Wittig-Reaktion).

Die nachfolgenden Synthesebeispiele dienen der näheren Erklärung der Erfindung.

**BEISPIEL 1**

(5Z,7E,22E)-(1S,3R,24R)-9,10-Seco-24a-homo-5,7,10(19),22-cholestatetraen-1,3,24-triol (7) und

**BEISPIEL 2**

(5Z,7E,22E)-(1S,3R,24S)-9,10-Seco-24a-homo-5,7,10(19),22-cholestatetraen-1,3,24-triol (8)

I) Isobutylcarbonylmethylentriphenylphosphoran 1

a) Brommethylisobutylketon

50 ml Isobutylmethylketon in 240 ml Methanol werden bei 0°C mit 20 ml Brom versetzt und noch 1,5 Stunden bei 10°C gerührt. Anschließend werden 360 ml Wasser zugesetzt und 16 Stunden bei Raumtemperatur gerührt. Die Reaktionsmischung wird mit gesättigter Kochsalzlösung versetzt, die organische Phase abgetrennt und die wäßrige Phase mit Ether extrahiert. Die vereinigten organischen Phasen werden mit 10%iger Natriumcarbonatlösung gewaschen und über Calciumchlorid getrocknet. Nach Filtration wird das Lösungsmittel verdampft und der Rückstand destilliert. 53,7 g Brommethylisobutylketon werden als farbloses Öl erhalten; $K_p^{15-20}$ 67-69°C.

b) Isobutylcarbonylmethyltriphenylphosphoniumbromid

53,6 g Brommethylisobutylketon werden zu 78,5 g Triphenylphosphin gegeben und die abgekühlte Reaktionsmischung in Methylenchlorid/Ether (1:2) umkristallisiert. Es werden 111,7 g Phosphoniumbromid vom Schmelzpunkt 244-245°C erhalten.

c) Isobutylcarbonylmethylentriphenylphosphoran 1

111,6 g Isobutylcarbonylmethyltriphenylphosphoniumbromid in 1500 ml Methylenchlorid werden mit 1500 ml 2N NaOH versetzt und 30 Minuten bei Raumtemperatur gerührt.Die organische Phase wird abgetrennt, mit Wasser gewaschen und über Natriumsulfat getrocknet. Nach Filtration und Verdampfung des Lösungsmittels wird der Rückstand in tert.-Butylmethylether umkristallisiert.

Es werden 72,2 g der Titelverbindung 1 vom Schmelzpunkt 120-121°C erhalten. Durch Variationen der Ketonkomponente im Reaktionsschritt a) lassen sich in analoger Weise weitere Phosphorane der Formel herstellen.

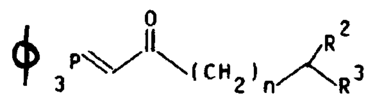

(VI)

II) (5E,7E,22E)-(1S,3R)-1,3-Bis-(tert.-butyldimethylsilyloxy-9,10-seco-24a-homo-5,7,10(19)-22-cholestatetraen-24-on 2

8,0 g (1S,3R)-Bis-(tert.-butyldimethylsilyloxy)-(20S)-formyl-9,10-secopregna-(5E,7E,10(19))-trien (A) (Calverley Tetrahedron 43, 4609 (1987)) und 12,0 g 1 werden in 46 ml Dimethylsulfoxid 6 Stunden bei 105°C unter Stickstoff gerührt. Anschließend wird die Reaktionsmischung bei Raumtemperatur mit Essigester verdünnt und mit Kochsalz-Lösung gewaschen. Die organische Phase wird über Natriumsulfat getrocknet und filtriert. Nach Entfernung des Lösungsmittels wird der Rückstand mit Toluol durch Kieselgel filtriert. Verdampfung des Lösungsmittels und Gradientenchromatographie (Toluol/Hexan (1:1) →Toluol) des Rückstandes an Kieselgel ergeben 3,6 g der Titelverbindung 2 als amorphen Feststoff.

III) (5E,7E)-(1S,3R,24R)-1,3-Bis-(tert.-butyldimethylsilyloxy)-9,10-seco-24a-homo-5,7,10(19),22-cholestatetraen-24-ol 3

(5E,7E,22E)-(1S,3R,24S)-1,3-Bis-(tert.-butyldimethylsilyloxy)-9,10-seco-24a-homo-5,7,10(19),22-cholestatetraen-24-ol 4

3,5 g der Verbindung 2 in 9 ml Tetrahydrofuran und 20,6 ml Methanol werden mit 20,6 ml einer 0,4 molaren methanolischen $CeCl_3 \cdot 7H_2O$-Lösung versetzt. Unter Stickstoff werden bei Eiskühlung 570 mg Natriumborhydrid portionsweise hinzugegeben. Die Suspension wird noch 40 Minuten bei Eiskühlung gerührt und dann in Eis/Kochsalz-Lösung gegeben. Die Wasserphase wird mit Essigester extrahiert, die organische Phase mit Wasser neutral gewaschen und über Natriumsulfat getrocknet. Filtration und Entfernung des Lösungsmittels ergeben 3,5 g Öl. Durch Chromatographie an Kieselgel mit Essigester/Hexan (1:9) werden 534 mg 3 und 692 mg 4 jeweils als kristallisierendes Öl erhalten.

IV) (5Z,7E,22E)-(1S,3R,24R)-1,3-Bis-(tert.-butyldimethylsilyloxy)-9,10-seco-24a-homo-5,7,10(19),22-cholestatetraen-24-ol 5

534 mg 3 werden in 75 ml Toluol gelöst und nach Zugabe von 89 mg Anthracen und 1 Tropfen Triethylamin

5 Minuten bei Raumtemperatur mit einer Quecksilberhochdrucklampe (Heraeus TQ 150) durch Pyrex-Glas bestrahlt. Die trübe Reaktionsmischung wird filtriert, eingeengt und der Rückstand mit Essigester/Hexan (1:9) an Kieselgel chromatographiert. Es werden 410 mg der Titelverbindung 5 als Öl erhalten.

(5Z,7E,22E)-(1S,3R,24S)-1,3-Bis-(tert.-butyldimethylsilyloxy)-9,10-seco-24a-homo-5,7,10(19),22-cholestatetraen-24-ol 6

Analog den Bedingungen zur Herstellung von 5 werden aus 680 mg der Verbindung 4 570 mg der Titelverbindung 6 als Öl erhalten.

**V) BEISPIEL 1**

(5Z,7E,22E)-(1S,3R,24R)-9,10-Seco-24a-homo-5,7,10(19),22-cholestatetraen-1,3,24-triol 7

200 mg der Verbindung 5 in 8,8 ml Tetrahydrofuran werden mit 1,5 ml einer 1molaren Lösung von Tetrabutylammoniumfluorid in Tetrahydrofuran 50 Minuten bei 60°C gehalten. Die abgekühlte Reaktionsmischung wird mit Essigester verdünnt und mit Natriumhydrogencarbonat-Lösung und Kochsalz-Lösung gewaschen. Die organische Phase wird mit Wasser neutral gewaschen und über Natriumsulfat getrocknet. Filtration und Verdampfung des Lösungsmittels ergeben 210 mg Öl als Rückstand. Durch Chromatographie an Kieselgel mit Essigester/Hexan (2:1) werden 124 mg der Titelverbindung 7 als amorpher Feststoff erhalten. UV(MeOH) $\lambda$=210($\varepsilon$=14720), 264(14240)

**BEISPIEL 2**

(5Z,7E,22E)-(1S,3R,24S)-9,10-Seco-24a-homo-5,7,10(19),22-cholestatetraen-1,3,24-triol 8

Unter den Bedingungen des Beispiels 1 werden aus 200 mg der Verbindung 6 88 mg der Titelverbindung 8 vom Schmelzpunkt 128-129°C erhalten.

**BEISPIEL 3**

(5Z,7E,22E)-(1S,3R,24S)-24-Cyclopropylmethyl-9,10-secochola-5,7,10(19),22-tetraen-1,3,24-triol (14)

I) (Cyclopropylmethyl)-(triphenylphosphoranyliden)-keton (9)

180 mg Lithiumchlorid und 540 mg Kupfer(I)chlorid werden unter Stickstoff in 9 ml Tetrahydrofuran 1,5 Stunden bei Raumtemperatur gerührt. Nach Abkühlung auf 10°C werden 15,0 g (Brommethyl)-(triphenylphosphoranyliden)-keton (M. Le Corre, C.R. Acad. Sc. (C) 273, 81 (1971)) in 225 ml Tetrahydrofuran zugesetzt und 30 Minuten gerührt. Anschließend werden 29,5 ml einer ca. 1,6 molaren Lösung von Cyclopropylmagnesiumbromid in Tetrahydrofuran (G.F. Reynolds et al., J. Org. Chem. 23, 1217 (1958)) zugetropft und 1,5 Stunden bei gleicher Temperatur gerührt. Zur Aufarbeitung gießt man die Reaktionsmischung in Eis/gesättigte Ammoniumchlorid-Lösung und extrahiert anschließend mit Essigsäureethylester. Die organische Phase wird über Natriumsulfat getrocknet und dann eingeengt. Durch Chromatographgie des festen Rückstandes an Kieselgel mit Essigsäureethylester/Aceton (3:1) werden 5,6 g der Titelverbindung 9 vom Schmelzpunkt 152°C erhalten.

II) (5E,7E,22E)-(1S,3R)-1,3-Bis-(tert.-butyldimethylsilyloxy)-24-cyclopropylmethyl-9,10-secochola-5,7,10(19),22-tetraen-24-on 10

Durchführung und Aufarbeitung wie unter II

Ansatz: 3,72 g der (20S)-Formylverbindung A<br>
5,6 g 9<br>
} in 21 ml Dimethylsulfoxid

Ausbeute: 2,2 g 10 als kristallisiertes Öl vom Fp.: 97-98°C.

III) (5E,7E,22E)-(1S,3R,24R)-1,3-Bis-(tert.-butyldimethylsilyloxy)-24-cyclopropyl-methyl-9,10-secochola-5,7,10(19),22-tetraen-24-ol 11 und

(5E,7E,22E)-(1S,3R,24S)-1,3-Bis-(tert.-butyldimethylsilyloxy)-24-cyclopropylmethyl-9,10-secochola-5,7,10(19),22-tetraen-24-ol 12

Durchführung und Aufarbeitung wie unter III, Beispiele 1/2

Ansatz: 2,2 g 10 in 5,8 ml THF und 13,0 ml Methanol
13,0 ml 0,4 molare $CeCl_3.7\ H_2O$-Lsg.
359 mg $NaBH_4$
2,22 g Öl als Rohprodukt

Ausbeute: 1,05 g 11 (mit 8 verunreinigt
330 mg 12 als Harz

In den nachfolgenden Reaktionen wird nur die weitere Umsetzung von 12 beschrieben; auch 11 läßt sich analog wie nachstehend beschrieben weiterverarbeiten.

IV) (5Z,7E,22E)-(1S,3R,24S)-1,3-Bis-(tert.-butyldimethylsilyloxy)-24-cyclopropyl-methyl-9,10-secochola-5,7,10(19),22-tetraen-24-ol 13

320 mg 12 werden in 45 ml Toluol gelöst und nach Zugabe von 54 mg Anthracen und 1 Tropfen Triethylamin 5 Minuten bei Raumtemperatur mit einer Quecksilberhochdrucklampe (Hereaus TQ 150) durch Pyrex-Glas bestrahlt. Die Reaktionsmischung wird eingeengt und der Rückstand (375 mg) - ein Gemisch aus 13 und Anthracen - direkt mit Tetrabutylammoniumfluorid umgesetzt.

**V) BEISPIEL 3**

(5Z,7E,22E)-(1S,3R,24S)-24-Cyclopropylmethyl-9,10-secochola-5,7,10(19),22-tetraen-1,3,24-triol 14

375 mg des Rückstandes von 13 in 14,2 ml Tetrahydrofuran werden mit 2,39 ml einer 1 molaren Lösung von Tetrabutylammoniumfluorid in Tetrahydrofuran unter Stickstoff 60 Minuten bei 60°C gehalten. Zur Aufarbeitung wird die abgekühlte Reaktionsmischung in kalte Natriumhydrogencarbonatlösung gegossen und anschließend mit Essigsäureethylether extrahiert. Nach Trocknung der organischen Phase über Natriumsulfat, Filtration und Verdampfung des Lösungsmittels werden 400 mg harzartiger Rückstand erhalten. Chromatographie an Kieselgel mit Essigsäureethylester/Hexan (2:1) ergeben 150 mg 14 vom Schmelzpunkt 137-139°C.

**BEISPIEL 4**

(5Z,7E,22E)-(1S,3R,24S)-9,10-Seco-24a,24b-dihomo-5,7,10(19),22-cholestatetraen-1,3,24-triol (16)

**I) Isoamylcarbonylmethylentriphenylphosphoran 15**

Die Titelverbindung 15 wird als Feststoff vom Schmelzpunkt 64-67°C aus Isoamylmethylketon analog dem beschriebenen Verfahren zur Darstellung von Isobutylcarbonylmethylentriphenylsphosphoran erhalten.

II) - V) Die Titelverbindung 16 wird analog der beschriebenen Sequenz II)-V) in Beispiel 1 aus 5,69 g (1S,3R)-Bis-(tert.-butyldimethylsilyloxy)-(20S)formyl-9,10-secopregna-(5E,7E,10(19))-trien A und 8,93 g Isoamylcarbonylmethylentriphenylphosphoran 15 als Feststoff vom Schmelzpunkt 119-120,5°C erhalten.

**BEISPIEL 5**

(5Z,7E,22E)-(1S,3R,24S)-24-Cyclopentylmethyl-9,10-secochola-5,7,10(19),22-tetraen-1,3,24-triol 18

**I) Cyclopentylmethylcarbonylmethylentriphenylphosphoran 17**

Die Titelverbindung 17 wird als kristallisiertes Öl vom Schmelzpunkt 84-87°C aus Cyclopentylaceton analog dem beschriebenen Verfahren zur Darstellung von Isobutylcarbonylmethylentriphenylphosphoran erhalten.

II) - V) Die Titelverbindung 18 wird analog der beschriebenen Sequenz II)-V) in Beispiel 1 aus 3,50 g (1S,3R)-Bis-(tert.-butyldimethylsilyloxy)-(20S)formyl-9,10-secopregna-(5E,7E,10(19))-trien A und 5,68 g 17

als Feststoff vom Schmelzpunkt 90-93°C erhalten.

**BEISPIEL 6**

(5Z,7E,22E)-(1S,3R,24R)-24-(2-Ethylbutyl)-9,10-secochola-5,7-10(19),22-tetraen-1,3,24-triol 25 und

**BEISPIEL 7**

(5Z,7E,22E)-(1S,3R,24S)-24-(2-Ethylbutyl)-9,10-secochola-5,7,10(19),22-tetraen-1,3,24-triol 26

**I) 4-Ethyl-pentanoylmethylentriphenylphosphoran 19**

a) Ethyl-2-buten-1(2)-carbonitril 19 a

170 g (2 Mol) Cyanessigsäure werden zusammen mit 10 g Ammoniumacetat und 10 g Essigsäure in 100 ml Benzol in einem 1 l Kolben vorgelegt. Dazu werden 172 g (2 Mol) Diethylaceton zugegeben und 18 Stunden ganz vorsichtig am Rückfluß mit einem Wasserabscheider gekocht. Nach dem Verdampfen von Benzol wird der Rückstand in 1000 ml 2 n HCl gegeben, mit Ether extrahiert, die organische Phase abgetrennt, mit gesättigter NaCl-Lösung gewaschen, über $Na_2SO_4$ getrocknet und eingeengt. Der Rückstand wird 2 mal bei 18 mm/Hg destilliert. Es werden 96,7 g 19 a als farbloses Öl erhalten.
Kp.66-68° C, IR ν(CN) 2240-2212 $cm^{-1}$.

b) Ethyl-2-butancarbonitril 19 b

96,7 g (886 mMol) 4-Ethyl-2-buten-1(2)-carbonitril 19 a werden mit Ethanol auf ein Volumen von 150 ml gebracht. Dazu werden 2 g Pd/Kohle 10%ig zugegeben und bei 50°C 7 Stunden hydriert. Das Reaktionsgemisch wird über Celite filtriert, eingeengt, mit 400 ml 2 n Cl aufgenommen, mit Ether extrahiert, getrocknet, eingeengt und im Vakuum destilliert. Man erhält 50,5 g Substanz 19 b als farbloses Öl.
$Kp^{6-8}$. 45°C, NMR(300MHz) [ δ ] 0,92(6H,t) 1,5 (5H,m) 2,34 (2H,d)

c) 4-Ethyl-2-hexanon 19 c

14,4 g (600 mMol) Mg-Späne werden mit 150 ml Diethylether in einem Dreihalskolben vorgelegt. Dazu werden 85,2 g (600 mMol) Methyljodid in 50 ml Ether zugetropft. Anschließend werden 100 ml Benzol zugegeben und ein Teil des Diethylethers abdestilliert. 33,3 g (300 mMol) Ethyl-2-butylcarbonitril 19 b werden zugegeben und 5 Stunden am Rückfluß gekocht. Das Reaktionsgemisch wird mit $NH_4Cl$-Lösung hydrolysiert, mit Ether extrahiert, mit Wasser gewaschen, über $Na_2SO_4$ getrocknet, eingeengt und im Vakuum über eine Vigreux-Kolonne destilliert. Man erhält 12,4 g 19 c als farbloses Öl.
$Kp^{16}$ 54°C, IR ν(C0) 1730 $cm^{-1}$
NMR [ δ ] 0,86 (6H,t) 1,30 (4H,m) 1,80 (1H,m) 2,13 (3H,s) 2,33 (2H,d)

d) Brommethyl-4-ethyl-pentylketon 19 d

12 g (93 mMol) 4-Ethyl-2-hexanon in 60 ml Methanol werden bei 0°C mit 14,8 g (93 mMol) Brom versetzt und noch 30 Minuten bei 0°C gerührt. Anschließend werden 100 ml Wasser unter Kühlung zugesetzt und 16 Stunden bei Raumtemperatur gerührt. Die Reaktionsmischung wird mit gesättigter Kochsalzlösung versetzt, mit Ether extrahiert, mit gesättigter $NaHCO_3$ gewaschen und Über $Na_2SO_4$ getrocknet. Nach Filtration wird das Lösungsmittel verdampft und der Rückstand destilliert. Es werden 13,4 g Brommethyl-4-ethylpentylketon 19 d erhalten.
$Kp^{12}$ 54°C
NMR (300 MHz) [ δ ] 0,86 (6H,t) 1,23 (4H,m) 1,86 (1H,m) 2,58 (2H,d) 3,88 (2H,s)

e) 4-Ethylpentanoylmethyltriphenylphosphoniumbromid 19 e

13,4 g (65 mMol) Brommethyl-4-ethylpentylketon 19 d werden zu 17,3 g (65 mMol) Triphenylphosphin gegeben. Nach Stehenlassen über Nacht werden 76 ml Methylenchlorid zugegeben und 30 Minuten am Rückfluß gekocht. Nach dem Abkühlen wird das Reaktionsgemisch mit 110 ml Ether versetzt und mit 80 ml Methylenchlorid/Ether (3:5) gewaschen. Man erhält 26,4 g des Phosphoniumbromids 19 e.

NMR (300 MHz) [ δ ] 0,78 (6H, t) 1,20 (4H,m) 1,77 (1H,m) 2,87 (2H,d) 5,7 (2H,d) 7,78 (15H,m)

f) 4-Ethylpentanoylmethylentriphenylphosphoran 19

26 g (55 mMol) 4-Ethylpentanoylmethyltriphenylphosphoniumbromid 19 e in 70 ml Methanol werden mit 5,04 g (60 mMol) Natriumhydrogencarbonat in 70 ml Wasser versetzt und 30 Minuten bei Raumtemperatur gerührt. Das Reaktionsgemisch wird in Wasser gegeben, mit Methylenchlorid extrahiert, die organische Phase abgetrennt, mit Wasser gewaschen, über $Na_2SO_4$ getrocknet und eingeengt. Der Rückstand wird mit Essigester verrührt. Es werden 18,8 g der Titelverbindung 19 erhalten.
NMR (300 MHz) [ δ ] 0,93 (6H,t) 1,52 (4H,m) 2,31 (1H,m) 2,48 (2H,d) 3,94 (1H,br d) 7,48 und 7,88 (15H,m)

**II) 5E,7E,22E)-(1S,3R)-1,3-Bis-(tert.-butyldimethylsilyloxy)-24-(2-ethylbutyl)-9,10-secochola-5,7,10(19),22-tetraen-24-on (20)**

3,4 g (5,9 mMol) der (20S) Formylverbindung A und 5,7 g (14,7 mMol) 19 werden in 100 ml Toluol über Nacht bei 80°C erwärmt. Anschließend wird das Reaktionsgemisch in Wasser gegeben, die organische Phase abgetrennt, mit gesättigter Kochsalzlösung gewaschen, über $Na_2SO_4$ getrocknet und eingeengt. Der ölige Rückstand wird an Kieselgel mit Toluol chromatographiert. Es werden 1,1 g der Verbindung 20 als Öl erhalten.
NMR (300 MHz) [ δ ] 0,53 (3H,S) 0,79 (24 H) 1,2 (3H,d) 4,17 (1H,m) 4,48 (1H,m) 4,81 (2H,d) 5,78 (1H,d) 5,98 (1 H,d) 6,39 (1H,d) 6,62 (1H,q)

**III) (5E,7E,22E)-(1S,3R,24R)-1,3-Bis-(tert.-butyldimethylsilyloxy)-24-(2-ethylbutyl)-9,10-secochola-5,7,10(19),22-tetraen-24-ol 21**

**(5E,7E,22E)-(1S,3R,24S)-1,3-Bis(tert.-butyldimethylsilyloxy)-24-(2-ethylbutyl)-9,10-secochola-5,7,10(19),22-tetraen-24-ol 22**

Durchführung und Aufarbeitung wie unter III, Beispiele 1/2
Ansatz: 1,1 g (1,6 mMol) 20 in 2,8 ml THF und 6,3 ml Methanol
920 mg (2,47 mMol) $CeCl_3$ x $7H_2O$ in 6,1 ml Methanol
263 mg (6,96 mMol) $NaBH_4$
Ausbeute: 340 mg 21 und 140 mg 22 als Öle.
21 und 22 NMR (300 MHz)
[ δ ] 0,50 (3H,S) 0,7-0,88 (24H) 4,05 (1H,m) 4,14 (1H,m) 4,48 (1H,m) 4,91 (2H,d) 5,32 (1H,dd) 5,47 (1H,dd) 5,77 (1H,d) 6,40 (1H,d)

**IV) (5Z,7E,22E)-(1S,3R,24R)-1,3-Bis-tert.-butyldimethylsilyloxy)-24-(2-ethylbutyl)-9,10-secochola-5,7,10(19)-tetraen-24-ol 23**

340 mg 21 werden in 60 ml Toluol gelöst und nach Zugabe von 53 mg (0,30 mMol) Anthracen und 1 Tropfen Triethylamin 5 Minuten bei Raumtemperatur mit einer Quecksilberhockdrucklampe (Heraus TQ 150) durch Pyrex-Glas bestrahlt. Die trübe Reaktionsmischung wird filtriert und eingeengt. Es werden 340 mg der Titelverbindung 23 erhalten.

**(5Z,7E,22E)-(1S,3R,24S)-1,3-Bis(tert.-butyldimethylsilylox)-24-(2-ethylbutyl)-9,10-secochola-5,7,10(19),22-tetraen-24-ol 24**

Analog den vorstehenden Bedingungen unter IV) werden aus 130 mg der Verbindung 22 130 mg der Titelverbindung 24 als Öl erhalten.

**V) (5Z,7E,22E)-(1S,3R,24R)-24-(2-Ethylbutyl)-9,10-secochola-5,7,10(19),22-tetraen-1,3,24-triol (25)**

340 mg der Verbindung 23 in 10 ml Tetrahydrofuran werden mit 2 ml 1 n Tetrabutylammoniumfluorid in Tetrahydrofuran 1 Stunde bei 55°C gehalten. Die abgekühlte Reaktionsmischung wird mit Essigester verdünnt und mit Natriumhydrogencarbonat-Lösung und Kochsalz-Lösung gewaschen. Die organische Phse wird mit Wasser neutralgewaschen, über Natriumsulfat getrocknet und eingeengt. Nach der Chromatographie an Kieselgel mit Essigester und Eindampfen wird der Rückstand mit Hexan aufgenommen und filtriert. Es werden 110 mg der Titelverbindung 25 als amorpher Feststoff erhalten. Fp.147-154°C.

(5Z,7E,22E)-(1S,3R,24S)-24-(2-Ethylbutyl)-9,10-secochola-5,7,10(19),22-tetraen-1,3,24-triol 26

Unter den Bedingungen zur Herstellung von 25 werden aus 130 mg der Verbindung 24 40 mg der Titelverbindung 26 vom Schmelzpunkt 135-138°C erhalten.

**Patentansprüche**

1. 24-Homo-Vitamin-D-Derivate der Formel I

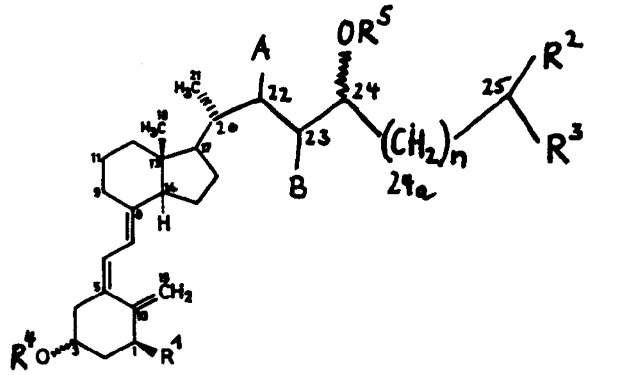

(I),

worin

| | |
|---|---|
| $R^1$ | ein Wasserstoffatom, eine Hydroxy- oder eine Acyloxygruppe mit 1 bis 8 Kohlenstoffatomen, |
| $R^2$ und $R^3$ | unabhängig voneinander eine 1 bis 4 Kohlenstoffatome aufweisende lineare oder verzweigte Alkylgruppe, eine Trifluormethylgruppe oder gemeinsam einen mit dem Kohlenstoffatom 25 gebildeten gesättigten carbocyclischen Ring mit 3 bis 9 Kohlenstoffatomen |
| $R^4$ und $R^5$ | unabhängig voneinander ein Wasserstoffatom oder eine 1 bis 8 Kohlenstoffatome aufweisende Acylgruppe und |
| A und B | jeweils ein Wasserstoffatom oder gemeinsam eine zweite Bindung bedeuten sowie |
| n | für 1, 2, 3, 4 oder 5 |

steht.

2. 24-Homo-Vitamin-D-Derivate nach Anspruch 1, worin $R^1$ für eine Hydroxygruppe steht.

3. 24-Homo-Vitamin-D-Derivate nach Anspruch 1, worin $R^4$ und $R^5$ jeweils für ein Wasserstoffatom stehen.

4. 24-Homo-Vitamin-D-Derivate nach Anspruch 1, worin $R^2$ und $R^3$ jeweils für eine Methyl- oder Ethylgruppe stehen.

5. 24-Homo-Vitamin-D-Derivate nach Anspruch 1, worin $R^2$ und $R^3$ gemeinsam mit dem Kohlenstoffatom 25 für einen Cyclopropyl-, Cyclopentyl- oder Cyclohexylring stehen.

6. 24-Homo-Vitamin-D-Derivate nach Anspruch 1, worin A und B gemeinsam für eine zweite Bindung stehen.

7. 24-Homo-Vitamin-D-Derivate nach Anspruch 1, worin n 1 ist.

8. (5Z,7E,22E)-(1S,3R,24R)-9,10-Seco-24a-homo-5,7,10(19),22-cholestatetraen-1,3,24-triol.
(5Z,7E,22E)-(1S,3R,24S)-9,10-Seco-24a-homo-5,7,10(19),22-cholestatetraen-1,3,24-triol.
(5Z,7E,22E)-(1S,3R,24S)-24-Cyclopropylmethyl-9,10-secochola-5,7,10,(19),22-tetraen-1,3,24-triol
(5Z,7E,22E)-(1S,3R,24S)-24-Cyclopentylmethyl-9,10-secochola-5,7,10(19),22-tetraen-1,3,24-triol
(5Z,7E,22E)-(1S,3R,24R)-24-(2-Ethylbutyl)-9,10-secochola-5,7-10(19),22-tetraen-1,3,24-triol
(5Z,7E,22E)-(1S,3R,24S)-24-(2-Ethylbutyl)-9,10-secochola-5,7,10(19),22-tetraen-1,3,24-triol

9. Verfahren zur Herstellung von 24-Homo-Vitamin-D-Derivaten der Formel I

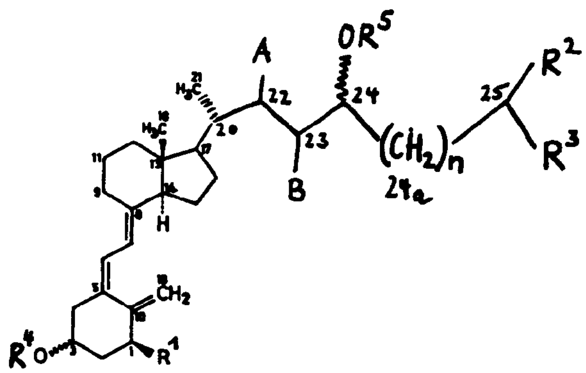

(I),

worin $R^1$, $R^2$, $R^3$, $R^4$ und $R^5$, A und B sowie n die in Anspruch 1 angegebene Bedeutung haben, dadurch gekennzeichnet, daß eine Verbindung der allgemeinen Formel IV

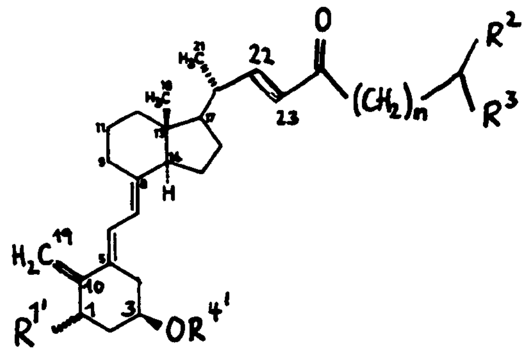

(IV),

worin
$R^{1'}$ ein Wasserstoffatom oder eine geschützte Hydroxygruppe und
$R^{4'}$ eine Hydroxyschutzgruppe bedeuten und
$R^2$ und $R^3$ sowie n die in Formel I angegebene Bedeutung haben, gegebenenfalls nach selektiver Hydrierung der 22,23-Doppelbindung, durch Reduktion der 24-Carbonylfunktion in eine Verbindung der allgemeinen Formel III

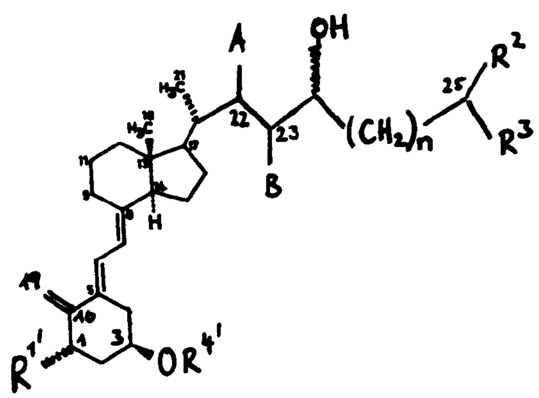

(III),

worin $R^{1'}$, $R^{4'}$, $R^2$ und $R^3$ sowie n die in Formel IV angegebene Bedeutung haben und
A und B entweder gemeinsam eine zweite Bindung oder jeweils ein Wasserstoffatom bedeuten
überführt,
eine Verbindung der allgemeinen Formel III durch Bestrahlung mit ultraviolettem Licht unter Umkehr der Stereoisomerie an der 5,6-Doppelbindung in eine Verbindung der allgemeinen Formel II

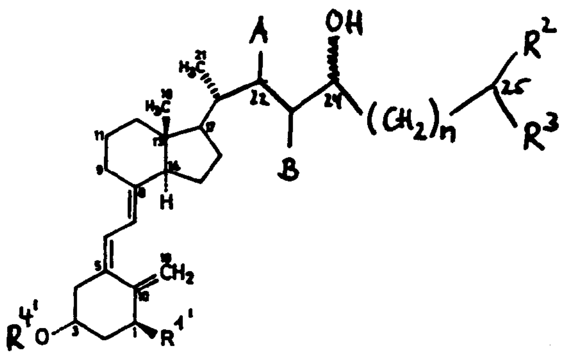

(II),

worin
$R^{1'}$, $R^{4'}$, $R^2$ und $R^3$, n sowie A und B die in Formel III angegebene Bedeutung haben,
umgewandelt
und diese anschließend durch Abspaltung vorhandener Hydroxyschutzgruppen und gegebenenfalls durch partielle oder vollständige Veresterung der Hydroxygruppen in eine Verbindung der allgemeinen Formel I überführt wird.

10. Pharmazeutische Präparate, dadurch gekennzeichnet, das sie mindestens eine Verbindung gemäß den Ansprüchen 1 bis 8 sowie einen pharmazeutisch verträglichen Träger enthalten.

11. Verwendung der Verbindungen gemäß den Ansprüchen 1 bis 8 zur Herstellung von Arzneimitteln.

## Claims

1. Derivatives of the 24-homo-vitamin D in accordance with formula I

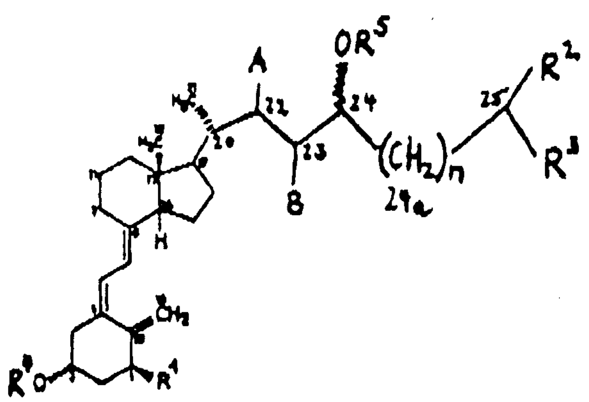

(I),

where

| | |
|---|---|
| $R^1$ | is a hydrogen atom, a hydroxy or an acyloxy group with 1 to 8 carbon atoms, |
| $R^2$ and $R^3$ | when independent of each other, are linear or branched alkyl radicals having 1 to 4 carbon atoms, or a trifluoromethyl group or, together, represent a saturated carbocyclic ring with 3 to 9 carbon atoms incorporating the carbon atom 25, |
| $R^4$ and $R^5$ | independent of each other. are a hydrogen atom or an acyl radical having 1 to 8 carbon atoms, and |
| A and B | each represent one hydrogen atom or, together, represent a second bond, |
| n | meaning 1, 2, 3, 4, or 5. |

2. Derivatives of the 24-homo-vitamin D in accordance with Claim 1, where $R^1$ represents a hydroxy radical.

3. Derivatives of the 24-homo-vitamin D in accordance with Claim 1, where $R^4$ and $R^5$ each represent a hydrogen atom.

4. Derivatives of the 24-homo-vitamin D in accordance with Claim 1, where $R^2$ and $R^3$ each represent a methyl or ethyl group.

5. Derivatives of the 24-homo-vitamin D in accordance with Claim 1, where $R^2$ and $R^3$ together with the carbon atom 25 represent a cyclopropyl, cyclopentyl or cyclohexyl ring.

6. Derivatives of the 24-homo-vitamin D in accordance with Claim 1, where A and B together represent a second bond.

7. Derivatives of the 24-homo-vitamin D in accordance with Claim 1, where n is 1.

8. (5Z,7E,22E)-(1S,3R,24R)-9,10-seco-24a-homo-5,7,10(19),22-cholestatetraene-1,3,24-triol.
(5Z,7E,22E)-(1S,3R,24S)-9,10-seco-24a-homo-5,7,10(19),22-cholestatetraene-1,3,24-triol.
(5Z,7E,22E)-(1S,3R,24S)-24-cyclopropylmethyl-9,10-secochola-5,7,10(19),22-tetraene-1,3,24-triol.
(5Z,7E,22E)-(1S,3R,24S)-24-cyclopentylmethyl-9,10-secochola-5,7,10(19),22-tetraene-1,3,24-triol.
(5Z,7E,22E)-(1S,3R,24R)-24-(2-ethylbutyl)-9,10-secochola-5,7-10(19),22-tetraene-1,3,24-triol.
(5Z,7E,22E)-(1S,3R,24S)-24-(2-ethylbutyl)-9,10-secochola-5,7,10(19),22-tetraene-1,3,24-triol.

9. Method for the manufacture of derivatives of the 24-homo-vitamin D in accordance with formula I

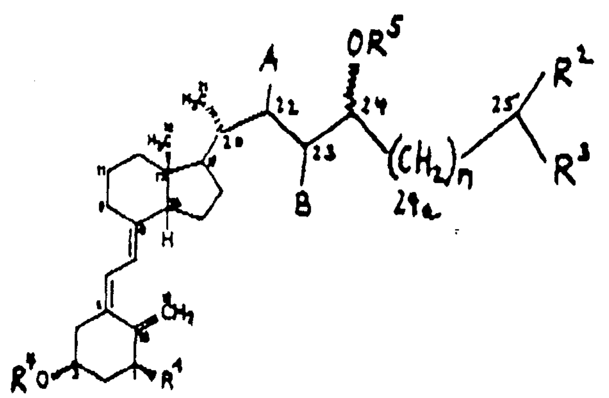

(I),

where $R^1$, $R^2$, $R^3$, $R^4$ and $R^5$, A and B as well as n have the meaning indicated in Claim 1. characterized in that a compound of the general formula IV

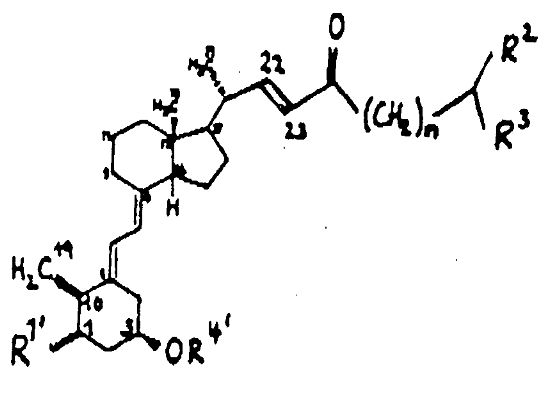

(IV),

where

| | |
|---|---|
| $R^{1'}$ | is a hydrogen atom or a protected hydroxy group, and |
| $R^{4'}$ | is a hydroxy protecting group, and |
| $R^2$ and $R^3$ | as well as n have the meaning indicated in formula I, |

is converted into a compound of the general formula III by reduction of the 24-carbonyl function - if necessary after selective hydration of the 22,23 double bond -

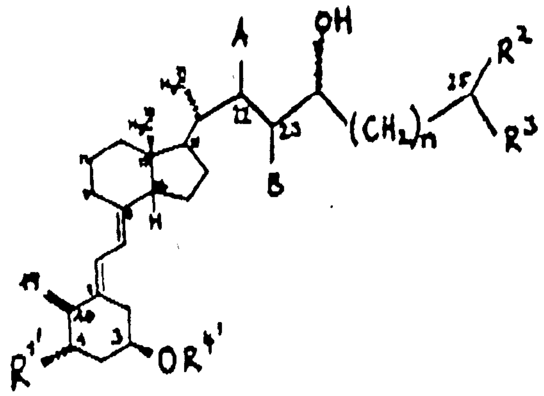

(III),

10. Pharmaceutical specialities, characterized in that they contain at least one compound in accordance with Claims 1 to 8 as well as one pharmaceutically tolerated carrier.

11. Use of the compounds in accordance with Claims 1 to 8 in the manufacture of medicines.

## Revendications

1. Derivés de la vitamine 24-homo-D suivant la formule I

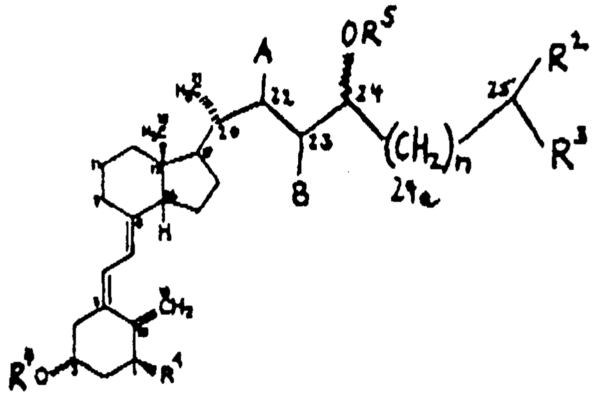

(I),

où

| | |
|---|---|
| $R^1$ | est un atome d'hydrogène, un groupe hydroxy ou un groupe acyloxy possédant 1 à 8 atomes de carbone, |
| $R^2$ et $R^3$ | où ils sont indépendents l'un de l'autre, sont des radicaux linéaires ou ramifiés d'alkyle possédant 1 à 4 atomes de cabone, ou un groupe de trifluorométhyl ou, ensemble, représentent un cycle carboxyle saturé possédant 3 à 9 atomes de carbone y inclus l'atome de carbone 25, |
| $R^4$ and $R^5$ | , indépendent l'un de l'autre, représentent chacun un atome de carbone ou un groupe acyle possédant 1 à 8 atomes de carbone, et |
| A and B | représentent chacun un atome d'hydrogène ou, ensemble, une deuxième liaison; |
| n | signifie 1, 2, 3, 4 ou 5. |

2. Derivés de la vitamine 24-homo-D suivant la revendication 1, où $R^1$ représente un groupe hydroxy.

3. Derivés de la vitamine 24-homo-D suivant la revendication 1, où $R^4$ et $R^5$ représentent chacun un atome d'hydrogène.

4. Derivés de la vitamine 24-homo-D suivant la revendication 1, où $R^2$ et $R^3$ représentent chacun un groupe méthyle ou éthyle.

5. Derivés de la vitamine 24-homo-D suivant la revendication 1, où $R^2$ et $R^3$ conjointement avec l'atome de carbone 25 représentent un cycle comme le cyclopropyle, cyclopentyle ou cyclohexyle.

6. Derivés de la vitamine 24-homo-D suivant la revendication 1, où A et B ensemble représentent une deuxième liaison.

7. Derivés de la vitamine 24-homo-D suivant la revendication 1, où n est 1.

8. (5Z,7E,22E)-(1S,3R,24R)-9,10-séco-24a-homo-5,7,10(19),22-cholestatetraène-1,3,24-triol.
(5Z,7E,22E)-(1S,3R,24S)-9,10-séco-24a-homo-5,7,10(19),22-cholestatetraène-1,3,24-triol.
(5Z,7E,22E)-(1S,3R,24S)-24-cyclopropylméthyl-9,10-sécochola-5,7,10(19),22-tetraène-1,3,24-triol,
(5Z,7E,22E)-(1S,3R,24S)-24-cyclopentylméthyl-9,10-sécochola-5,7,10(19),22-tetraène-1,3,24-triol.
(5Z,7E,22E)-(1S,3R,24R)-24-(2-éthylbutyl)-9,10-sécochola-5,7-10(19),22-tetraène-1,3,24-triol.
(5Z,7E,22E)-(1S,3R,24S)-24-(2-éthylbutyl)-9,10-sécochola-5,7,10(19),22-tetraène-1,3,24-triol.

9. Procédé pour la fabrication de dérivés de la vitamine 24-homo-D suivant la formule I

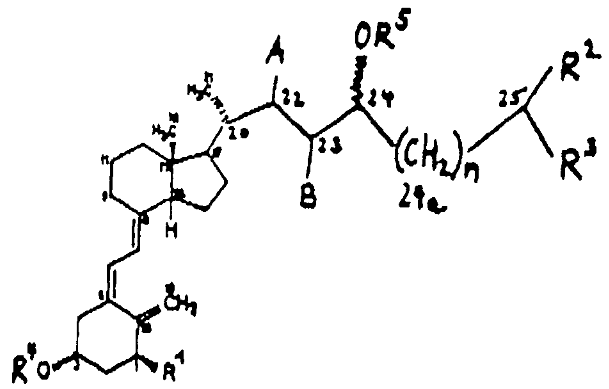

(I),

où $R^1$, $R^2$, $R^3$, $R^4$ et $R^5$, A et B ainsi que n ont la signification indiquée dans la revendication 1, caractérisé en ce qu'un composé de la formule générale IV

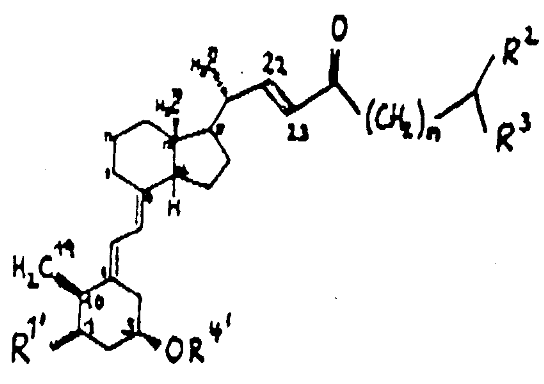

(IV),

où
$R^{1'}$ est un atome d'hydrogène ou un groupe hydroxy protégé et
$R^{4'}$ est und groupe hydroxy de protection et
$R^2$ et $R^3$ ainsi que n ont la signification indiquée dans la formule I,
est transformé par réduction de la fonction 24-carbonyle et le cas échéant, après hydratation sélective de la liaison double 22,23, en un composé de la formule générale III

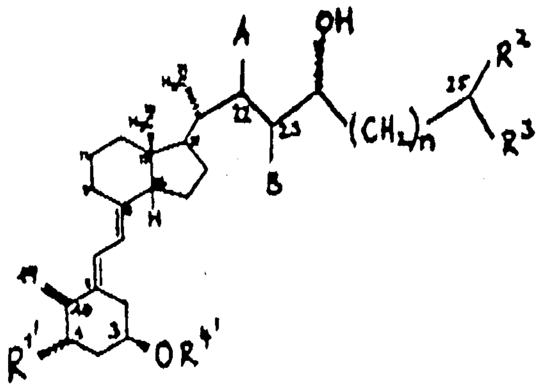

(III),

où $R^{1'}$, $R^{4'}$, $R^2$ et $R^3$ ainsi que n ont la signification indiquée dans la formule IV, et A et B représentent soit ensemble une deuxième liaison soit chacun pour soi un atome d'hydrogène,
et qu'un composé de la formule générale III est transformé par exposition à la lumière ultraviolette et sous inversion de la stéréoisomérie de la liaison double 5,6 en un composé de la formule générale II

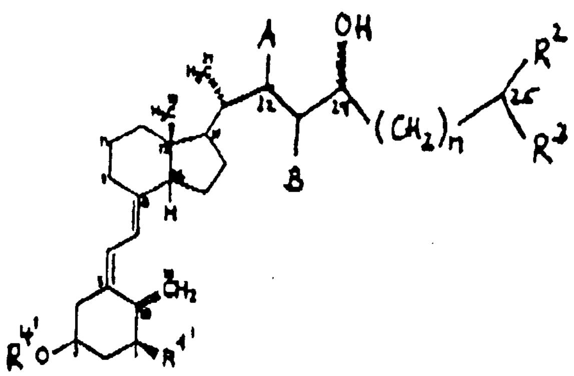

(II),

où
$R^{1'}$, $R^{4'}$, $R^2$ et $R^3$ , n ainsi que A et B ont la signification indiquée dans la formule III,
et que ce composé est ensuite transformé par élimination de groupes de protection hydroxy éxistant et, le cas échéant, par estérification partielle ou complète des groupes hydroxy en un composé de la formule générale I.

**10.** Préparations pharmaceutiques, caractérisées en ce qu'elles contiennent au moins un des composés suivant les revendications 1 à 8 ainsi qu'un véhicule pharmaceutiquement tolérable.

**11.** Utilisation des composés suivant les revendications 1 à 8 dans la fabrication de médicaments.